# EUROPEAN PATENT APPLICATION

(11) **EP 3 363 466 A1**
(43) Date of publication of application: **22.08.2018**
(21) Application number: 16855801.3
(22) Date of filing: 17.10.2016
(51) Int. Cl.: A61K 49/06, A61K 49/10

(54) **METHOD FOR PREPARING CONTRAST AGENT FOR MAGNETIC RESONANCE IMAGING**

(30) Priority: 16.10.2015 KR 20150144793
(71) Applicant: Enzychem Lifesciences Corporation, Daejon 34051 (KR)
(72) Inventor: CHOI, Kyung Seok, Daejeon 34062 (KR); SOHN, Ki Young, Seoul 07264 (KR); LEE, Jae Yong, Yongin-si Gyeonggi-do 16927 (KR)
(74) Representative: Byrne, Declan
(86) International application number: PCT/KR2016/011605
(87) International publication number: WO 2017/065584

(57) **Abstract**

The present invention relates to a method for preparing a contrast agent for magnetic resonance imaging (MRI) and, more particularly, to a method for preparing a contrast agent for magnetic resonance imaging, which has improved stability by minimizing side effects by removing unreacted substances generated during a preparation process. The preparation method comprises the steps of: reacting a lanthanide with a macrocyclic chelate to form a complex-containing aqueous solution; exchanging ions using one or more resins among a cation-exchange resin, a chelate resin and an anion-exchange resin in order to remove free lanthanide and unreacted macrocyclic chelate from the complex-containing aqueous solution; controlling pH of the complex aqueous solution in a neutral range; and removing germs from the complex aqueous solution.

## Description

### [Technical Field]

The present invention relates to a method for preparing a contrast agent for magnetic resonance imaging (MRI), and more particularly, to a method for preparing a contrast agent for magnetic resonance imaging, which includes removing unreacted substances generated during a preparation process, thus to minimize side effects, thereby improving stability.

### [Background Art]

Imaging techniques for diagnosis including, for example, magnetic resonance imaging, X-ray imaging, nuclear medicine radiation imaging, UV/visible light/infrared-ray imaging and ultrasonic wave imaging, have been used for diagnosis in several years. Contrast agents generally used in such a field may include, for example, organic molecules, metal ions, salts or chelates, iron particles or marked peptide, protein, polymer or liposome, etc.

Especially, a contrast agent for magnetic resonance imaging ('MRI'), as an example of the above-described contrast agents, may include gadopentetate dimeglumine (trade name: Magnevist) which has firstly been commercially available in 1988, as well as gadodiamide (trade name: Omiscan), gadoteridol (trade name: Prohance), gadoterate meglumine (trade name: Dotarem), gadobutrol (trade name: Gadovist), etc., which are now commercially available in the market. These agents may image blood vessels with an ionic compound, thus to be used for diverse diagnoses of cardiovascular disease, vascular tumor, etc., and further may provide important information on blood vessels in various transplant operations or other surgeries.

Since the MRI contrast agent as described above is used in a small amount, there is relatively little side effect due to osmotic pressure and viscosity. However, it is broadly known that unreacted substances such as free gadolinium, unreacted macrocyclic chelate, etc. remain in a product to cause side effects. Therefore, it is necessary to minimize the side effects, thereby improving stability.

Techniques relating thereto have been described in detail in known documents including, for example, Korean Patent Laid-Open Publication Nos. 2012-0033769 and 2009-0123171, Korean Patent Registration No. 1466602, Japanese Patent Laid-Open Publication No. 2001-521545, and the like.

### [Disclosure]

### [Technical Problem]

In consideration of the above-mentioned circumstances, it is an object of the present invention to provide a method for preparing a contrast agent for magnetic resonance imaging (hereinafter referred to as an 'MRI'), which may minimize a content of unreacted substances to reduce toxicity affecting the human body, thereby improving stability.

### [Technical Solution]

In order to achieve the above object, there is provided a method for preparing a contrast agent for MRI, which includes: reacting a lanthanide compound with a macrocyclic chelate to form a complex-containing aqueous solution; conducting ion-exchange using at least one resin among a cation-exchange resin, a chelate resin and an anion-exchange resin, in order to remove unreacted substances from the complex-containing aqueous solution; controlling pH of the complex-containing aqueous solution in a neutral range thereof; and removing germs from the complex-containing aqueous solution.

### [Advantageous Effects]

The method for preparing a contrast agent for MRI according to the present invention may decrease a content of free lanthanide metal ions or unreacted macrocyclic chelate generated during formulation of the contrast agent, so as to minimize side effects caused by the above components, for example, nephrotic toxicity.

### [Best Mode]

Hereinafter, the present invention will be described in detail. Prior to this, terms or words used in the specification and claims should not be construed as limited to a conventional or lexical meaning. Instead, based on a principle that an inventor may desirably define the concept of terms or words to explain his/her invention by means of the most preferable method, the terms or words should be construed as the meanings and concepts in compliance with technical spirits of the present invention.

It should be noted that a reaction temperature, a reaction time, the number of processing works, a content of resin and a pH range, etc., are defined in the most desirable range in the method for preparing a contrast agent according to the present invention. Further, all % denotes % by w/v unless otherwise specified.

The method for preparing a contrast agent for MRI according to the present invention may include: reacting lanthanide ions with a macrocyclic chelate to form a complex-containing aqueous solution; conducting ion-exchange using at least one resin among a cation-exchange resin, a chelate resin and an anion-exchange resin, in order to remove unreacted substances from the complex-containing aqueous solution; controlling pH of the complex-containing aqueous solution in a neutral range thereof; and removing germs from the complex-containing aqueous solution.

According to the above preparation method, removing the unreacted substances possibly remaining during formulation of a contrast agent, for example, removing free lanthanide ions or macrocyclic chelate may minimize a residual amount thereof, which in turn, can greatly reduce toxicity affecting the human body.

According to one embodiment of the present invention, the above preparation method may include, firstly, reacting a lanthanide compound with a macrocyclic chelate in an aqueous solvent to form a complex aqueous solution.

The reaction of forming a complex may be carried out at 50°C or higher, for example, 50 to 100°C, 70 to 90°C or 75 to 80°C for 1 to 10 hours or 5 to 6 hours.

In this case, the lanthanide compound used herein may include a gadolinium compound, for example, in a form of oxide, chloride, hydroxide, etc. For example, Gd₂O₃ is preferably used.

According to one embodiment of the present invention, the macrocyclic chelate used in the complex forming reaction may include, for example, DOTA (1,4,7,10-tetraazacyclo-dodecane-N-N,N',N",N'"-tetraacetic acid represented by Formula 1 below, BT-DO₃A (1,4,7-tris(carboxymethyl)-10,11-hydroxymethyl-2,3-dihydroxypropyl-1,4,7,10-tetraazacyclododecane).

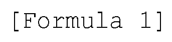

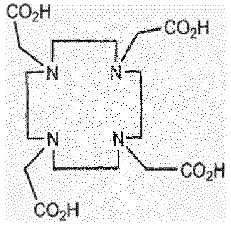

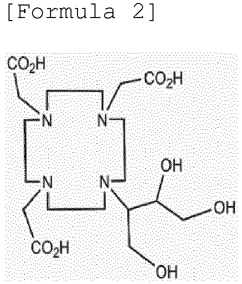

In this case, the lanthanide compound and the macrocyclic chelate may be used in a stoichiometric content, for example, in a molar ratio of 0.9 to 1.1 : 0.9 to 1.1. Preferably, using excess lanthanide compound in a stoichiometric content relative to the macrocyclic chelate so that the unreacted chelate compound is substantially not present therein. In other words, if providing the lanthanide compound in a sufficient stoichiometric content relative to the macrocyclic chelate, most of the macrocyclic chelates may be combined with lanthanide ions to form a complex, so that an unreacted macrocyclic chelate is substantially not present in the complex-containing aqueous solution which is a product consequently obtained by the reaction.

Such a condition, in which the macrocyclic chelate is substantially not present in the complex-containing aqueous solution, means that a content of the macrocyclic chelate is very close to 0 mole in the aqueous solution, and may include a case in which the macrocyclic chelate is absolutely not present therein.

As described above, in order to use a stoichiometric excess lanthanide compound relative to the macrocyclic chelate, it is necessary to accurately assaying purities of starting materials, that is, the macrocyclic chelate and the lanthanide compounds. For this purpose, each of the components is subjected to quantitative analysis of purity at least three times, for example, three to five times, followed by calculating an average value from the measured values. With reference to the calculated average value, a content of the starting materials may be exactly quantified. In this case, the quantitative analysis used herein may include any one of conventional methods, so long as it is used in the related art without particular limitation thereof. For example, methods such as titration, HPLC, ICP, and AA may be used.

Based on the result of the quantitative analysis described above, the lanthanide compound may be used in an excess content relative to a content of the macrocyclic chelate, for example, in a content of about 0.50 to 1.0 equivalent or about 0.51 to 0.6 equivalent relative to the macrocyclic chelate.

In a case in which lanthanide and the macrocyclic chelate compound are combined with each other using any conventional method known in the art, it is difficult to control a content of free lanthanide or macrocyclic chelate. Further, a process of repeating quantitative analysis twice or three times to determine contents of the macrocyclic chelate and free lanthanide, and introducing these compounds again to regulate the contents thereof should be employed. This process is quite complicated and encounters a problem of requiring a long time. Further, when adding meglumine, calcobutrol, trometamol, etc. As an excipient during preparation of a final product, contents of the above two compounds are altered and thus should be adjusted again.

Meanwhile, in the present invention, the content of each of the macrocyclic chelate and free lanthanide is controlled to reach 0 mole. Accordingly, these compounds are selectively and conveniently added at the final stage and thus can achieve an advantage of very easily manufacturing a final product. Further, the present invention may make it easier to manufacture the final product by using a chelate resin, a cation-exchange resin or anion-exchange resin. Furthermore, using Dotarem, Gadovist, etc. as a contrast agent also allows a novel production process to have difference, easiness, economical advantage, convenience in process, etc., as compared to the conventional manufacturing methods.

With regard to the complex formed by the above-described reaction, when using gadolinium and DOTA as the lanthanide and the macrocyclic chelate, respectively, the complex may be gadoterate represented by Formula 3 below. When using BT-DO₃A as the macrocyclic chelate, the complex may be gadobutrol represented by Formula 4 below.

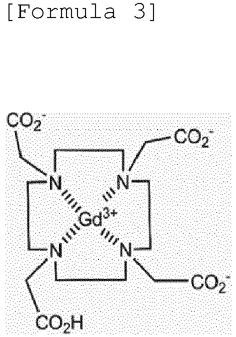

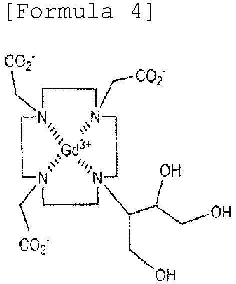

Then, the complex aqueous solution containing the complex obtained by the reaction as described above may necessarily include unreacted substances after the reaction, and these unreacted substances may act as a cause of inducing side effects. For this reason, a residual amount of the unreacted substances is required to be minimized. In particular, when using the stoichiometric excess lanthanide compound, the unreacted macrocyclic chelate compound is not present therein. However, free lanthanide ions necessarily remain in the complex aqueous solution. The present invention uses an ion-exchange resin to remove this residue. That is, the free lanthanide ions or others which remain in the complex-containing aqueous solution may be removed using the ion-exchange resin, so that these free lanthanide ions are substantially not present in the complex-containing aqueous solution.

A condition, in which the free lanthanide ion is substantially not present in the complex-containing aqueous solution, means that a content of the ions in the aqueous solution is very close to 0 mole, and may include a case in which the free lanthanide ion is absolutely not present therein.

The ion-exchange resin used herein may include a cation-exchange resin and/or an anion-exchange resin, a chelate resin, etc. For example, the chelate resin alone; a combination of the anion-exchange resin and the chelate resin; a combination of the cation-exchange resin and the chelate resin; a combination of the cation-exchange resin, the anion-exchange resin and the chelate resin, etc. may be used. However, any resin known so long as it is known in the related art may be used without particular limitation thereof.

The cation-exchange resin used herein may include a strong cation-exchange resin or weak cation-exchange resin. For example, the strong cation-exchange resin may include styrene-based gel type and porous type resins having a sulfonic acid group as an exchange group, such as SK1B, PK216, CGS, CG8, IR118, IR120, HCR-S, C120E, C100, S100, IR122, IR252, etc., while the weak cation-exchange resin may include methacrylate- and acryl-based porous type resin having a carboxylic acid group as an exchange group, such as PC200, MWC-1, WACG, IRC76, IRC86, MAC-3, C104, CNP-80, etc.

The anion-exchange resin used herein may include a strong anion-exchange resin or weak anion-exchange resin. For example, the strong anion-exchange resin may include a styrene-based gel type resin having an alkyl ammonium group as an exchange group, such as SAR10, SAR11, SA10A, SA20A, PA412, SBG1, SBG2, IRA402, IRA400, IR410, IRA900, IRA910, SBR, SAR, MSA-1C, A400, A600, A200, A300, M500, M600, MP600, etc., while the weak anion-exchange resin may include acryl-based and styrene-based resins having secondary amine and tertiary amine functional groups, respectively, as an exchange group, such as DIAION WA10, DIAION WA20, DIAION WA30, WBMP, IRA93, IRA92, IRA60, MWA-1, M-63, A100, A103, MP62, AP49, etc. Further, the chelate resin may include styrene-based and epoxy-based resins, such as DIAION CR11, DIAION CR20, Eporous MX8c, Eporous Z-7, Eporous K-1, Eporous K-3, Eporous K-6, PS400, PS410, PS460, PS470, IRC-718, IRC 748, IRC 747, S930, S940, S920, S910, CR11, XZ95843, XZ87480, XZ95844, TP207, TP260, TP214, SIR-300, SIR-500, SIR-200, etc.

The above-described ion-exchange resins may include commercially available products, for example, which are manufactured by Mitsubishi, Resintech, Rohm & Haas, Amberite, Dow, Purolite, Bayer lewatit, etc.

The above-described ion-exchange resins may be used in proportion to a content of the above-described raw materials, that is, lanthanide or macrocyclic chelate. As an alternative method, it is possible to conduct quantitative analysis of a content of free lanthanide or unreacted macrocyclic chelate which remains in the complex aqueous solution, then calculate an exchange capacity of the ion-exchange resin corresponding to the analyzed content and use the calculated capacity. For example, after analyzing a content of the unreacted macrocyclic chelate, an exchange capacity of the anion-exchange resin relative to moles may be calculated and used. Otherwise, after analyzing a content of the free lanthanide ions, an exchange capacity of the cation-exchange resin or chelate resin relative to moles may be calculated and used.

In this case, a reaction time for ion-exchange may depend upon the analyzed result. For example, the reaction may be conducted for 1 to 10 hours or 2 to 5 hours. Further, an ion-exchange reaction may be performed by introducing the cation-exchange resin or both of the chelate resin and the anion-exchange resin at the same time.

In the ion-exchange process as described above, the ion-exchange resin may be used in a ratio of 0.1 to 1.0 equivalent or more relative to the exchange capacity. Further, if necessary, the content of the ion-exchange resin may be controlled according to the exchange time and an exchange rate.

The ion-exchange process may be conducted only one time or may be repeatedly conducted in several stages. For example, the ion-exchange process may repeatedly proceed 2 to 5 times in order to minimize a residual amount of the unreacted substances described above.

The ion-exchange stage or the residual amount analysis stage may be conducted at 40°C or less, for example, in a range of 0 to 40°C. Within this range, an ion-exchange performance may be sufficiently expressed.

By the ion-exchange process as described above, the free lanthanide ions or unreacted macrocyclic chelates which remain in the complex-containing aqueous solution are removed, thus to minimize the contents thereof, and the ion-exchange resin may be removed by filtration.

After the ion-exchange stage as described above, if necessary, the complex may react with meglumine, calcobutrol or trometamol.

For example, in a case in which the complex is gadoterate, 0.9 to 1.1 equivalents of meglumine is added to and reacted with the complex, resulting in formation of gadoterate meglumine represented by Formula 5 below (trade name: Dotarem).

Further, for gadobutrol represented by Formula 6 below, Gadovist (trade name) may be formed using gadoterate and trometamol represented by Formula 7 below as an excipient. Likewise, calcobutrol represented by Formula 8 below may be formed using trometamol as an excipient.

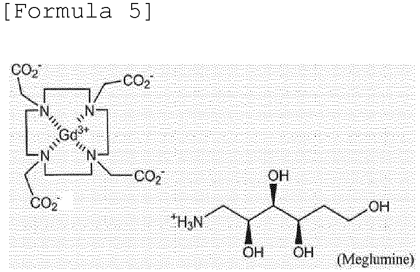

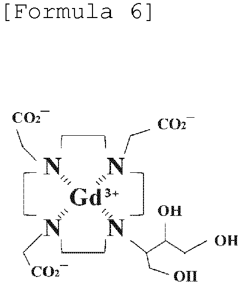

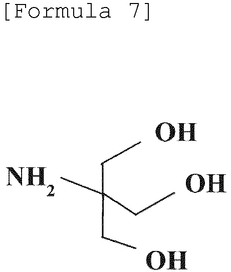

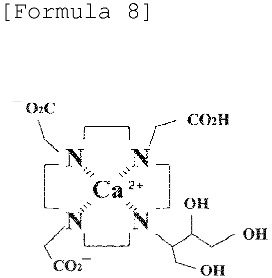

Following the ion-exchange stage and optionally the meglumine addition reaction, pH of the complex aqueous solution is controlled to be in a neutral range thereof. Such pH control may be conducted to obtain a pH similar to that of human blood, thereby preventing side effects. The pH control may be conducted using inorganic acid such as hydrochloric acid, or inorganic base such as sodium hydroxide, thereby controlling so as to be a neutral range, for example, a range of pH 6.5 to 8.0 or pH 6.8 to 7.4.

After the pH control process, a content of the free macrocyclic chelate in the aqueous solution is measured. This is a process for easily and ultimately controlling the contents of the free lanthanide ions and free macrocyclic chelate so as to reduce an error due to the analysis.

After the pH control process as described above, it is possible to further add the lanthanide compound and the macrocyclic chelate thereto. This addition may be done to control the contents of the macrocyclic chelate and the free lanthanide in manufacturing a final product according to instructions of a manufacturer or under permitted conditions. Herein, the macrocyclic chelate and the lanthanide compounds may be added in an additional content of 0.001 to 0.005 (mol/mol)% and/or 0.001 to 0.005 (mol/mol)%, respectively.

As such, reasons of additionally introducing the macrocyclic chelate and the free lanthanide compounds after preparation and then setting in a desired range are not only to reduce the analysis error but also to prevent degradation of the lanthanide chelate compound, so as to prevent a permissible content of the manufactured medicine from being reduced during an expiration date of the medicine and ensure stability of the product.

Following the above process, a process of removing germs and filtration may be further conducted. Such a germ removal and filtration process may include any conventional method known in the related art without particular limitation thereof. If necessary, additional sterilization may be further conduced in a high-pressure closed vessel such as an autoclave. Such a sterilization process may be conducted by any conventional method known in the related art without particular limitation thereof.

In recent years, for health examination and in vivo tumor diagnosis, using the contrast agent for MRI is gradually increased, such that stability of the contrast agent becomes more significant. In this regard, a contrast agent obtained by the method for preparing a contrast agent according to the present invention may greatly reduce toxicity expressed after injecting the same into the human body, thereby minimizing additional side effects and allowing to conduct a safe examination.

Hereinafter, Examples will be described to more concretely understand the present invention. However, it will be apparent to those skilled in the art that various modifications and alterations of such examples may be possible as defined by the appended claims, and the scope of the present invention is duly not limited to the following examples. Such examples are provided for completely explaining the present invention to persons having ordinary knowledge and skills in the related art.

### Example 1

After adding 1.005 kg (2.52 moles) of DOTA and 0.468 kg (1.29 moles) of Gd₂O₃ to 4.0ℓ of water for injection, a reaction was performed at 75 to 80°C for 5 hours to form a gadolinium complex, that is, gadoterate. After completing the reaction, the temperature was reduced to 25°C, and 20 ml of a chelate exchange resin, IRC-718, and 40 ml of an anion-exchange resin, SAR10 were added to the reaction, followed by agitation for 2 hours. Thereafter, a sample was taken from the reaction product and subjected to analysis of a residual amount of free gadolinium and unreacted macrocyclic chelate (DOTA) through titration and HPLC methods, respectively.

As a result of the analysis, 0.001 (mol/mol)% of free gadolinium and 0.01 (mol/mol)% of unreacted residual macrocyclic chelate (DOTA) were detected.

After filtering the above-used chelate and anion-exchange resins, these compounds were washed with 1.0ℓ of water for injection, and then, mixed with the filtered product. Then, 0.475 kg of meglumine was added to the product and agitated for 2 hours, and 2 (w/w)% of NaOH was further added to control pH to 7.0, followed by removing filtering germs, thereby preparing gadoterate meglumine as a contrast agent.

### Example 2

After dissolving 1.512 kg of gadobutrol with 99.7% purity containing 0.01 (mol/mol)% of free gadolinium and 0.05 (mol/mol)% of BT-DO₃A in 2.0ℓ of water for injection, 20 ml of a cation-exchange resin, IR120, and 40 ml of an anion-exchange resin, IRA67, were further added thereto, followed by agitating the same at 25°C for 3 hours.

After filtering the ion-exchange resin, the ion-exchange resin was washed with 0.5ℓ of water for injection, and then, mixed with the filtered product to obtain a whole solution. The whole solution was subjected to germ removal and filtration. Herein, a content of free gadolinium was 0.001 (mol/mol)%, while a content of BT-DO₃A (1,4,7-tris(carboxymethyl)-10-11-hydroxymethyl-2,3-dihydroxypropyl-1,4,7,10-tetraazacyclododecane) without the gadolinium complex was 0.01 (mol/mol)%. Then, 1.283 g of calcobutrol was added as an excipient, and 3.028 g of trometamol was further added while pH was controlled to 7.0 using 2.0 ml of 3N-HCl.

### Comparative Example 1

As a result of measuring residual amounts of free gadolinium and macrocyclic chelate in commercially available Dotarem, these amounts were 0.01 (mol/mol)% and 0.05 (mol/mol)%, respectively.

### Comparative Example 2

As a result of measuring residual amounts of free gadolinium and macrocyclic chelate in commercially available Gadovist, these amounts were 0.008 (mol/mol)% and 0.05 (mol/mol)%, respectively.

### Example 3

DOTA and Gd₂O₃ used as starting materials were subjected to purity analysis through HPLC and ICP methods three times. Then, an average value of the analyzed purities for each of the above materials was set as follows:
DOTA purity = 99.95 (w/w)%; and Gd₂O₃ purity = 99.99 (w/w) %.

1010.050 g of DOTA and 468.047 g of Gd₂O₃ were added to 4.0L of water for injection, and 1.395 g of Gd₂O₃ corresponding to 0.3 (mol/mol)% was further added, followed by reacting the mixture at 75°C for 6 hours to synthesize gadoterate. After then, the product was cooled to 25°C, and a content of free DOTA was measured by HPLC method. The measured content was 0 (mol/mol)%.

Subsequently, a small amount of chelate resin, Chelex 100 (bioRad), was added three times, so that a concentration of free gadolinium (Gd³⁺) was 0 (mol/mol)%. The chelate resin, that is, Chelex 100 was subjected to filtration and washed with 1.0ℓ of water for injection. Then, 475 g of meglumine was added to the solution, followed by agitating the mixture for 2 hours and then controlling pH to 7.2 with 2 (w/w)%NaOH. Following this, 2.02 g of free DOTA corresponding to 0.002 (mol/mol)% was added and agitated for 2 hours, followed by removing germs and filtration, thereby preparing gadoterate meglumine as a contrast agent.

Contents of residual free gadolinium and residual macrocyclic chelates described in Examples 1, 2, 3 and Comparative Examples 1 and 2, respectively, are shown in Table 1 below.

**[TABLE 1]**

| Item | Residual free gadolinium (mol/mol) % | Residual macrocyclic chelate (mol/mol) % |
|---|---|---|
| Comparative Example 1 | 0.01 | 0.05 |
| Comparative Example 2 | 0.008 | 0.05 |
| Example 1 | 0.001 | 0.01 |
| Example 2 | 0.001 | 0.01 |
| Example 3 | 0.000 | 0.00 |

According to Examples 1 and 2, it could be seen that the free gadolinium ions and macrocyclic chelate are removed by the cation-exchange resin, or the chelate resin and the anion-exchange resin in the preparation of gadoterate meglumine and gadobutrol, and therefore, residual amounts of the above two materials are maintained at a lower level than those measured in Comparative Examples 1 and 2, respectively. Further, according to Example 3, it is possible to exclude the unreacted macrocyclic chelate using excess gadolinium oxide, and then, remove the free gadolinium ions using the resins. Consequently, the contrast agent according to the present invention may have minimized toxicity affecting the human body, thereby being industrially applicable and easily applied to actual production.

## Claims

1. A method for preparing a contrast agent for magnetic resonance imaging (MRI), comprising:
conducting quantitative analysis of purities of Gd₂O₃ and DOTA (1,4,7,10-tetraazacyclo-dodecane-N,N',N",N'"-tetraacetic acid) as starting materials;
reacting DOTA with stoichiometric excess Gd₂O₃ to form a gadoterate-containing aqueous solution so that a concentration of unreacted DOTA is 0.000 (mol/mol)%;
conducting ion-exchange using a chelate resin in the gadoterate-containing aqueous solution so that a concentration of free gadolinium ions is 0.00 (mol/mol)%;
controlling pH of the gadoterate-containing aqueous solution in a neutral range;
adding meglumine to the gadoterate-containing aqueous solution and reacting the meglumine and the gadoterate-containing aqueous solution so as to combine gadoterate with the meglumine;
further adding DOTA or Gd₂O₃ to the gadoterate meglumine-containing aqueous solution; and
removing germs from the gadoterate meglumine-containing aqueous solution.
